# EUROPÄISCHE PATENTSCHRIFT

(11) **EP 1 104 419 B1**
(45) Veröffentlichungstag und Bekanntmachung des Hinweises auf die Patenterteilung: **29.05.2002**
(21) Anmeldenummer: 99938298.9
(22) Anmeldetag: 20.07.1999
(51) Int. Cl.: C07D 417/06, A61K 31/425

(54) **N-SUBSTITUIERTE AZABICYCLOHEPTAN-DERIVATE, IHRE HERSTELLUNG UND VERWENDUNG**
N-SUBSTITUTED AZABICYCLOHEPTANE DERIVATIVES, PRODUCTION AND USE THEREOF
DERIVES A SUBSTITUTION N D'AZACYCLOHEPTANE, PREPARATION ET UTILISATION DESDITS UTILISES

(30) Priorität: 12.08.1998 DE 19836406
(43) Veröffentlichungstag der Anmeldung: 06.06.2001
(73) Patentinhaber: BASF AKTIENGESELLSCHAFT, 67056 Ludwigshafen (DE)
(72) Erfinder: STEINER, Gerd, D-67281 Kirchheim (DE); HÖGER, Thomas, D-68535 Edingen-Neckarhausen (DE); UNGER, Liliane, D-67065 Ludwigshafen (DE); TESCHENDORF, Hans-Jürgen, D-67373 Dudenhofen (DE); JUCHELKA, Frieder, D-69181 Leimen (DE)
(74) Vertreter: Grünecker, Kinkeldey, Stockmair & Schwanhäusser Anwaltssozietät
(86) Internationale Anmeldenummer: EP9905164
(87) Internationale Veröffentlichungsnummer: WO0009501

(56) Entgegenhaltungen:
- WO-A-94/00431
- WO-A-94/00458
- WO-A-95/15312
- WO-A-96/04245
- WO-A-96/04272

## Beschreibung

Die Erfindung betrifft neue N-substituierte Azabicycloheptan-Derivate, und ihre Verwendung zur Bekämpfung von Krankheiten.

Exo-6-Phenyl-3-azabicyclo[3.2.0]heptan-Derivate besitzen interessante Eigenschaften als potentielle Neuroleptika (WO 94/00458, WO 95/15312). Hierbei spielen die beobachteten hohen Affinitäten zu D₄- und 5-HT₂-Rezeptoren eine besondere Rolle.

Die interessanteste Substanz aus obigen Verbindungsklassen mit hoher D₄/5-HT_{2A}-Affinität und guter Selektivität versus D₂ ist (+)-(1S,5R,6S)-exo-3-[2-[6-(4-Fluor-phenyl)-3-azabicyclo-[3.2.0]heptan-3-yl]-ethyl]-1H,3H-chinazolin-2,4-dion (= Substanz A), die ein potentielles Neuroleptikum darstellt. Die Dosierung der Substanz A ist allerdings nach oben begrenzt durch auftretende Verlängerungen des QT-Intervalls am Herz-EKG.

Es wurden nun Substanzen mit besseren Eigenschaften gefunden.

Gegenstand der Erfindung sind N-substituierte 3-Azabicyclo-[3.2.0]heptan-Derivate der Formel I 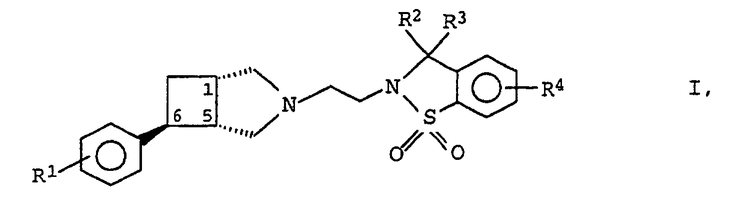 worin
- R¹: Fluor oder Chlor bedeutet,
- R² und R³: Wasserstoff oder C₁-C₃-Alkyl bedeuten und
- R⁴: Chlor, Methyl, Nitro oder Amino ist,
und deren Salze mit physiologisch verträglichen Säuren.

Bevorzugt sind die Verbindungen, in denen
- R¹: Chlor vorzugsweise in der p-Stellung,
- R²: Wasserstoff oder Methyl,
- R³: Wasserstoff oder Methyl und
- R⁴: Wasserstoff
bedeuten.

Die folgenden Verbindungen sind als besonders bevorzugt zu nennen:
(+)-(1S,5R,6S)-exo-2-[2-[6-(4-Chlor-phenyl)-3-azabicyclo-[3.2.0]heptan-3-yl]-ethyl]-3,3-dimethyl-2,3-dihydro-1,2-benzisothiazol-1,1-dioxid,
(+)-(1S,5R,6S)-exo-2-[2-[6-(4-Chlor-phenyl)-3-azabicyclo-[3.2.0]heptan-3-yl]-ethyl]-2,3-dihydro-1,2-benzisothiazol-1,1-dioxid und
(+)-(1S,5R,6S)-exo-2-[2-[6-(4-Fluor-phenyl)-3-azabicyclo-[3.2.0]heptan-3-yl]-ethyl]-2,3-dihydro-1,2-benzisothiazol-1,1-dioxid.

Die erfindungsgemäßen Verbindungen der Formel I lassen sich herstellen, indem man eine Verbindung der Formel II 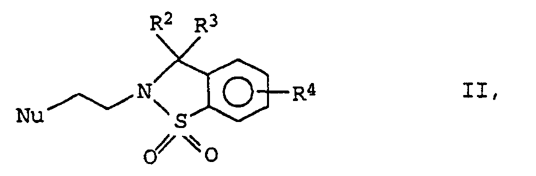 in der R², R³ und R⁴ die oben angegebenen Bedeutungen haben und Nu eine nukleofuge Abgangsgruppe darstellt, mit einem 3-Azabicyclo-[3.2.0]heptan-Derivat der Formel III als (+)-(1S,5R,exo-6S)-Enantiomeres 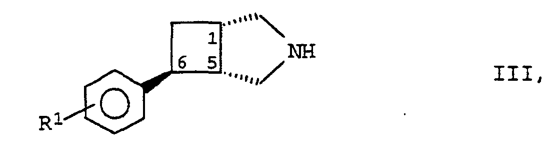 worin R¹ die oben angegebene Bedeutung hat, umsetzt und die so erhaltene Verbindung gegebenenfalls in das Säureadditionssalz einer physiologisch verträglichen Säure überführt.

Als nukleofuge Abgangsgruppe für Nu kommen vorzugsweise Halogenatome, insbesondere Brom oder Chlor, in Betracht.

Die Umsetzung erfolgt zweckmäßig in Gegenwart einer inerten Base, wie Triethylamin oder Kaliumcarbonat, als säurebindendes Mittel in einem inerten Lösungsmittel, wie einem cyclischen gesättigten Ether, insbesondere Tetrahydrofuran oder Dioxan, oder einem Benzolkohlenwasserstoff, wie Toluol oder Xylol.

Die Umsetzung erfolgt in der Regel bei Temperaturen von 20 bis 150°C, insbesondere von 80 bis 140°C, und ist im allgemeinen innerhalb von 1 bis 10 Stunden beendet.

Die erfindungsgemäßen Verbindungen der Formel I können entweder durch Umkristallisation aus den üblichen organischen Lösungsmitteln, bevorzugt aus einem niederen Alkohol, wie Ethanol, umkristallisiert oder durch Säulenchromatographie gereinigt werden.

Die freien 3-Azabicyclo[3.2.0]heptan-Derivate der Formel I können in üblicher Weise in das Säureadditionssalz einer pharmakologisch verträglichen Säure überführt werden, vorzugsweise durch Versetzen einer Lösung mit einem Äquivalent der entsprechenden Saure. Pharmazeutisch verträgliche Säuren sind beispielsweise Salzsäure, Phosphorsäure, Schwefelsäure, Methansulfonsäure, Amidosulfonsäure, Maleinsäure, Fumarsäure, Oxalsäure, Weinsäure oder Zitronensäure.

Die erfindungsgemäßen Verbindungen weisen wertvolle pharmakologische Eigenschaften auf. Sie können als Neuroleptika (insbesondere atypische), Antidepressiva, Sedativa, Hypnotika, ZNS-Protektiva oder Mittel zur Behandlung der Cocain-Abhängigkeit Verwendung finden. Mehrere der genannten Wirkungsqualitäten können kombiniert bei einer erfindungsgemäßen Verbindung auftreten.

Die Substanzen sind insbesondere durch eine sehr hohe und selektive Affinität zum Dopamin D₄- und Serotonin 2A-Rezeptor charakterisiert.

Die am Modell des Meerschweinchen-Papillarmuskels gemessenen Verlängerungen des QT-Intervalls sind vernachlässigbar gering. Deshalb sind die neuen Substanzen auch in hohen Dosierungen gut verträglich.

Die Erfindung betrifft dementsprechend auch die Verwendung der neuen Verbindungen bei der Bekämpfung von Krankheiten.

Die erfindungsgemäßen Verbindungen können in üblicher Weise oral oder parenteral, intravenös oder intramuskulär verabfolgt werden.

Die Dosierung hängt vom Alter, Zustand und Gewicht des Patienten sowie von der Applikationsart ab. In der Regel beträgt die tägliche Wirkstoffdosis zwischen etwa 1 und 100 mg/kg Körpergewicht bei oraler Gabe und zwischen 0,1 und 10 mg/kg Körpergewicht bei parenteraler Gabe.

Die neuen Verbindungen können in gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et. al: Pharmazeutische Technologie, Thieme-Verlag, Stuttgart, 1978). Die so erhaltenen Applikationsformen enthalten den Wirkstoff normalerweise in einer Menge von 1 bis 99 Gew.-%.

Die als Ausgangsstoffe für die Synthese der erfindungsgemäßen Verbindungen benötigten Substanzen der Formel II und III sind bekannt (WO 94/00458; Heterocycles 40 (1), 319-330 (1995), Chimia 1990, 44, 120) oder lassen sich gemäß den in der Literatur beschriebenen Herstellmethoden aus analogen Ausgangsmaterialien synthetisieren.

### Die folgenden Beispiele dienen zur Erläuterung der Erfindung:

### A Herstellung der Ausgangsmaterialien

a) 2,3-Dihydro-1,2-benzisothiazol-1,1-dioxid
   Zu 7,1 g (187 mM) Lithiumaluminiumhydrid in 400 ml absolutem Tetrahydrofuran gab man portionsweise während 90 min 25,3 g (138 mM) Saccharin unter gutem Rühren unter Stickstoff hinzu; dabei hielt man die Temperatur durch Eiskühlung auf Raumtemperatur. Nach Rühren über Nacht kühlte man den Ansatz im Eisbad und tropfte unter gutem Rühren vorsichtig Wasser und anschließend 10 %ige Schwefelsäure hinzu. Nach Absaugen der ausgefallenen Hydroxide und Nachwaschen mit THF engte man das Filtrat ein, verteilte den Rückstand zwischen Methylenchlorid und Wasser, stellte mit 10 %iger Schwefelsäure sauer und wusch anschließend die organische Phase mit Natriumcarbonat-Lösung durch. Nach Trocknen mit Natriumsulfat und Filtrieren engte man die organische Phase ein. Man isolierte 12,0 g (52 %) Produkt in genügender Reinheit.
b) Die Herstellung von 3,3-Dimethyl-2,3-dihydro-1,2-benzisothiazol-1,1-dioxid erfolgte in literaturbekannter Weise (K. Auer, E. Hungerbühler, R.W. Lang Chimia 1990, 44, 120). Analog wurden 3,3-Diethyl-2,3-dihydro-1,2-benzisothiazol-1,1-dioxid (Fp.: 174°C), 3,3-Dimethyl-6-nitro-2,3-dihydro-1,2-benzisothiazol-1,1-dioxid (Fp.: 187°) und 3,3-Dimethyl-4-chlor-2,3-dihydro-1,2-benzisothiazol-1,1-dioxid erhalten.
c) 2-(2-Chlor-ethyl)-3,3-dimethyl-2,3-dihydro-1,2-benzisothiazol-1,1-dioxid
   2,5 g (12,7 mM) 3,3-Dimethyl-2,3-dihydro-1,2-benzisothiazol-1,1-dioxid in 50 ml 1,2-Dichlorethan wurden mit 2,1 g (32 mM) KOH-Pulver 88 %ig und 250 mg Benzyl-triethyl-ammoniumchlorid versetzt und 1 h am Rückfluß gekocht. Nach dem Abkühlen verteilte man den Ansatz zwischen Eiswasser und Methylenchlorid, stellte mit Salzsäure schwach sauer und trennte die organische Phase ab. Nach Trocknen mit Natriumsulfat und Einengen der organischen Phase isolierte man 3,2 g (97 %) Produkt als Öl in genügender Reinheit.
   In analoger Weise lassen sich 2-(2-Chlor-ethyl)-3,3-dimethyl-6-nitro-2,3-dihydro-1,2-benzisothiazol-1,1-dioxid, 2-(2-Chlor-ethyl)-3,3-diethyl-2,3-dihydro-1,2-benzisothiazol-1,1-dioxid und 2-(2-Chlor-ethyl)-4-chlor-3,3-dimethyl-2,3-dihydro-1,2-benzisothiazol-1,1-dioxid herstellen.
d) (+)-(1S,5R,6S)-exo-6-(4-Chlor-phenyl)-3-azabicyclo-[3.2.0]heptan
   Die Isolierung des (+)-Enantiomeren wurde gemäß der Vorschrift in Heterocycles 40 (1), 326 (1995) vorgenommen.
e) (+)-(1S,5R,6S)-exo-[3-(2-Chlor)-ethyl]-6-(4-chlorphenyl)-3-azabicyclo[3.2.0]heptan
   10,0 g (48,2 mM) (+)-(1S,5R,6S)-exo-6-(4-Chlor-phenyl)-3-azabicyclo[3.2.0]heptan in 200 ml Tetrahydrofuran wurden mit 7,3 g (50 mM) 1-Brom-2-chlorethan und 3,5 g (25 mM) fein pulverisiertem Kaliumcarbonat versetzt und 15 h am Rückfluß gekocht. Anschließend engte man den Ansatz am Rotationsverdampfer ein und nahm den Rückstand in 200 ml Methyl-tert.-butylether auf. Nach Waschen der organischen Phase mit Wasser bei pH 10 extrahierte man die wäßrige Phase mit Methyl-tert.-butylether nach. Die vereinigten organischen Phasen wurden nach dem Trocknen mit Natriumsulfat eingeengt. Das Rohprodukt reinigte man durch Flash-Chromatographie (Kieselgel, Laufmittel Essigester/n-Heptan 1/1). Man isolierte 6,7 g (52 %) Produkt als Öl mit [α]_{D} = + 91,7° (EtOH).
   In analoger Weise stellte man (+)-(1S,5R,6S)-exo-[3-(2-Chlor)-ethyl]-6-(4-fluor-phenyl)-3-azabicyclo[3.2.0]-heptan her.

### Herstellung der Endprodukte

### Beispiel 1

### (+)-(1S,5R,6S)-exo-2-[2-[6-(4-Chlor-phenyl)-3-azabicyclo-[3.2.0]heptan-3-yl]-ethyl]-3,3-dimethyl-2,3-dihydro-1,2-benzisothiazol-1,1-dioxid x HCl

3,0 g (14,5 mM) (+)-(1S,5R,6S)-exo-6-(4-Chlor-phenyl)-3-azabicyclo-[3.2.0]heptan in 60 ml Xylol wurden mit 3,75 g (14,5 mM) 2-(2-Chlor-ethyl)-3,3-dimethyl-2,3-dihydro-1,2-benzisothiazol-1,1-dioxid und 2,0 g (14,5 mM) fein pulverisiertem Kaliumcarbonat versetzt und 7 h am Rückfluß gekocht. Anschließend engte man am Rotationsverdampfer ein, verteilte den Rückstand zwischen Wasser und Methylenchlorid bei pH 10. Die wäßrige Phase extrahierte man nochmals mit Methylenchlorid und engte darauf die vereinigten organischen Phasen ein. Das Rohprodukt reinigte man durch Säulenchromatographie (Kieselgel, Laufmittel Methylenchlorid/Methanol 98/2. Man isolierte 4,2 g (69 %) Produkt als Öl, das in 200 ml Ether gelöst und mit etherischer HCl in das Hydrochlorid (Smp. 230 bis 232°C) übergeführt wurde. [α]_{D} = + 60,9° (EtOH)

| Elementaranalyse C₂₃H₂₇N₂O₂SCl x HCl | | | |
|---|---|---|---|
| Berechnet | C 59,10 | H 6,04 | N 5,99 |
| Gefunden | C 59,3 | H 6,3 | N 5,7 |

### Beispiel 2

### (+)-(1S,5R,6S)-exo-2-[2-[6-(4-Chlor-phenyl)-3-azabicyclo-[3.2.0]heptan-3-yl]-ethyl]-2,3-dihydro-1,2-benzisothiazol-1,1-dioxid x HCl

2,0 g (11,8 mM) 2,3-Dihydro-1,2-benzisothiazol-1,1-dioxid in 30 ml DMF wurden mit 360 mg (12,0 mM) Natriumhydrid 80 %ig versetzt und 2 h bei 90 bis 100°C Badtemperatur nachgerührt. Nach dem Abkühlen gab man 3,2 g (11,8 mM) (+)-(1S,5R,6S)-exo-[3-(2-Chlor)-ethyl]-6-(4-chlor-phenyl)-3-azabicyclo[3.2.0]-heptan hinzu und rührte 2 h bei 100°C Badtemperatur nach. Nach dem Abkühlen verteilte man den Ansatz zwischen Methyl-tert.-butylether und Wasser bei pH 10 und extrahierte die wäßrige Phase nochmals mit Methyl-tert.-butylether. Die organischen Phasen wurden vereinigt und eingeengt. Das Rohprodukt reinigte man durch Säulenchromatographie (Kieselgel, Laufmittel Methylenchlorid/Methanol 98/2). Man isolierte 4,5 g (95 %) Produkt als Öl ([α]_{D} = + 69,9°; EtOH), das in 200 ml Ether gelöst und mit etherischer HCl in das Hydrochlorid (Smp. 240 bis 242°C) übergeführt wurde.

| Elementaranalyse C₂₁H₂₃N₂O₂SCl x HCl | | | | |
|---|---|---|---|---|
| Berechnet | C 57,40 | H 5,51 | N 6,38 | Cl 16,14 |
| Gefunden | C 57,1 | H 5,5 | N 6,2 | Cl 16,0 |

### Analog Beispiel 1 und 2 wurden hergestellt:

3. (+)-(1S,5R,6S)-exo-2-[2-[6-(4-Fluor-phenyl)-3-azabicyclo-[3.2.0]heptan-3-yl]-ethyl]-3,3-dimethyl-2,3-dihydro-1,2-benzisothiazol-1,1-dioxid, Smp. 113 bis 115°C
4. (+)-(1S,5R,6S)-exo-2-[2-[6-(4-Fluor-phenyl)-3-azabicyclo-[3.2.0]heptan-3-yl]-ethyl]-3,3-diethyl-2,3-dihydro-1,2-benzisothiazol-1,1-dioxid x HCl x H₂O, Smp. 77 bis 79°C
5. (+)-(1S,5R,6S)-exo-2-[2-[6-(4-Fluor-phenyl)-3-azabicyclo-[3.2.0]heptan-3-yl]-ethyl]-2,3-dihydro-1,2-benzisothiazol-1.1-dioxid x HCl, Smp. 234 bis 236°C, [α]_{D} = + 67,1° (EtOH)
6. (+)-(1S,5R,6S)-exo-2-[2-[6-(4-Chlor-phenyl)-3-azabicyclo-[3.2.0]heptan-3-yl]-ethyl]-3,3-dimethyl-4-chlor-2,3-dihydro-1,2-benzisothiazol-1,1-dioxid x HCl, Smp. 225 bis 227°C

## Patentansprüche

1. N-substituierte 3-Azabicyclo[3.2.0]heptan-Derivate der Formel I 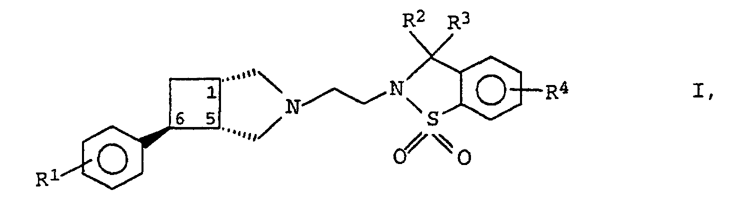 worin
R¹ Fluor oder Chlor bedeutet,
R² und R³ Wasserstoff oder C₁-C₃-Alkyl bedeuten und
R⁴ Chlor, Methyl, Nitro oder Amino ist,
und deren Salze mit physiologisch verträglichen Säuren.

2. Verbindungen der Formel I gemäß Anspruch 1 zur Verwendung bei der Bekämpfung von Krankheiten.

## Claims

1. N-substituted 3-azabicyclo[3.2.0]heptane derivatives of the formula I 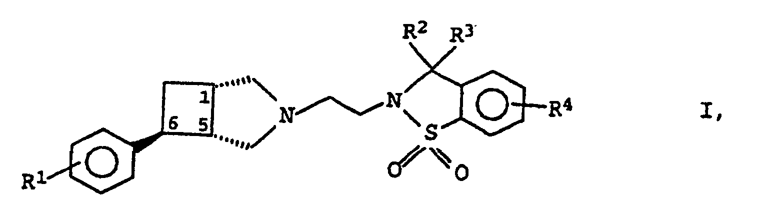 wherein
R¹ denotes fluorine or chlorine
R² and R³ denote hydrogen or C₁-C₃-alkyl, and
R⁴ is chlorine, methyl, nitro or amino,
and their salts with physiologically compatible acids.

2. Compounds of the formula I according to claim 1 for use in the treatment of medical conditions.

## Revendications

1. Dérivés substitués à l'azote du 3-azabicyclo[3.2.0]heptane de formule I 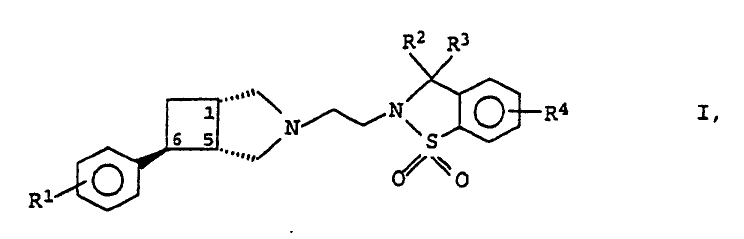 dans laquelle
R¹ représente le fluor ou le chlore,
R² et R³ représentent l'hydrogène ou des groupes alkyle en C1-C3 et
R⁴ représente le chlore, un groupe méthyle, nitro ou amino,
et leurs sels d'acides acceptables pour l'usage pharmaceutique.

2. Composés de formule I de la revendication 1 pour l'utilisation dans le traitement de maladies.
